# EUROPEAN PATENT APPLICATION

(11) **EP 3 058 874 A1**
(43) Date of publication of application: **24.08.2016**
(21) Application number: 13895526.5
(22) Date of filing: 17.10.2013
(51) Int. Cl.: A61B 8/00, G01N 29/24

(54) **ULTRASONIC PROBE HAVING VIBRATION GENERATING FUNCTION AND ULTRASONIC DIAGNOSTIC APPARATUS COMPRISING SAME**

(71) Applicant: Alpinion Medical Systems Co., Ltd., Seoul 152-848 (KR)
(72) Inventor: LEE, Su-Sung, Yongin-si Gyeonggi-do 448-140 (KR); SON, Keon-Ho, Seongnam-si Gyeonggi-do 463-440 (KR)
(74) Representative: Groth & Co. KB
(86) International application number: PCT/KR2013/009308
(87) International publication number: WO 2015/056823

(57) **Abstract**

The present invention relates to an ultrasonic probe having a vibration generating function, and an ultrasonic diagnostic apparatus comprising the same. The ultrasonic probe comprises a probe body; a transducer; and a vibration generating unit. The transducer is fixed to one end of the probe body, has a contact portion in contact with the surface of a test subject, and transmits and receives ultrasonic signals. The vibration generating unit is embedded in the probe body, and compresses and decompresses the surface of the test subject by the transducer by vibrating the probe body while the transducer is in contact with the surface of the test subject.

## Description

### Technical Field

The following description relates to an ultrasonic probe that uses elastography to acquire information about an image of internal tissues of an object, and an ultrasonic diagnostic apparatus having the same.

### Background Art

An ultrasonic diagnostic apparatus is an apparatus that uses an ultrasonic probe to obtain image information about internal tissues of an object by transmitting ultrasonic signals to the internal tissues and to receive ultrasonic signals reflected from the boundary of tissues of different acoustic impedance. The ultrasonic diagnostic apparatus may generate two-dimensional brightness mode (B-mode) images by displaying reflection coefficients reflected from internal tissues of an object as spots on a screen.

Meanwhile, it is a challenge to detect abnormal tissues, such as to cancer tissues and tumor tissues, using B-mode images, because difference in reflection coefficients among the abnormal tissues is not greater than that of normal tissues. To solve this drawback, elasticity imaging methods have been introduced. For example, in a case where tissues are transformed in response to an external force of equal magnitude, abnormal tissues, such as cancer tissues and tumor tissues, are relatively less transformed, but normal tissues are considerably transformed. The elasticity imaging methods are imaging methods using this characteristic. If elasticity of tissues are displayed in an image using elasticity imaging method, it may help to precisely detect abnormal tissues, such as cancer tissues and tumor tissues.

Among elastic imaging methods, there is a method for generating an image by causing an image to be transformed in response to an external force applied on a surface of an object, and then measuring strain that occurs due to difference between elasticity coefficients of tissues. In this case, a diagnostician periodically presses the surface of the object by manually applying a static force or vibration to an ultrasonic probe

However, if a diagnostician presses the surface of the object by manually applying a static force or vibration to an ultrasonic probe, magnitude of the force or time periods of vibration may not be constant. Thus, obtained images may have reduced repeatability and reproducibility, and it is hard to obtain uniform elasticity images..

### Technical Problem

The following description relates to an ultrasonic probe that may obtain uniform elasticity images and improve repeatability and reproducibility of the obtained elasticity images, and an ultrasonic diagnostic apparatus having the same.

### Technical Solution

In one general aspect, there is provided an ultrasonic probe including: a probe body; a transducer fixed to one end portion of the probe body and configured to transmit and receive an ultrasonic signal, have a contact portion to be in contact with a surface of an object; and a vibration generator accommodated in the probe body and configured to vibrates the probe body when the transducer is in contact with the surface of the object, thereby allowing the transducer to compress and decompress the surface of the object.

In another general aspect, there is provided an ultrasonic diagnostic apparatus including: an ultrasonic probe including: a probe body; a transducer fixed to one end portion of the probe body and configured to transmit and receive an ultrasonic signal and have a contact portion to be in contact with a surface of an object; and a vibration generator accommodated in the probe body and configured to vibrates the probe body when the transducer is in contact with the surface of the object, thereby allowing the transducer to compress and decompress the surface of the object, a controller configured to generate an elasticity image by obtaining an ultrasonic signals received from the transducer at a time when driving the vibration generator to compress or decompress the surface of the object.

### Advantageous Effects

According to the present disclosure, a vibration generator accommodated in a probe body vibrates an ultrasonic probe, so that a surface of an object is compressed or decompressed. Thus, uniform elasticity images may be obtained in real time, compared to a case where a diagnostician manually applies a static force or vibration on an ultrasonic probe. In addition, it may possible to improve repeatability, reproducibility, and quality of elasticity images.

### Description of Drawings

FIG. 1 is a diagram illustrating a configuration of an ultrasonic diagnostic apparatus according to an exemplary embodiment;
FIG. 2 is a perspective view illustrating an example of an ultrasonic probe with respect to FIG. 1;
FIG. 3 is an exploded perspective view illustrating a vibration generator placed in a probe body with respect to FIG. 2;
FIGS. 4 and 5 are diagrams for explaining a procedure for compressing or decompressing a surface of an object using an ultrasonic probe with respect to FIG. 2;
FIG. 6 is a cross-sectional view illustrating an example of a transducer with respect to FIG. 2;
FIG. 7 is a partial sectional view illustrating another example of a vibration generator; and
FIG. 8 is a partial sectional view illustrating an example in which a surface of an object is compressed by a vibration generator with respect to FIG. 7.

### Mode for Invention

The invention is described more fully hereinafter with reference to the accompanying drawings, in which exemplary embodiments of the invention are shown. This invention may, however, be embodied in many different forms and should not be construed as limited to the embodiments set forth herein. Rather, these embodiments are provided so that this disclosure is thorough, and will fully convey the scope of the invention to those skilled in the art. In the drawings, the size and relative sizes of layers and regions may be exaggerated for clarity. Like reference numerals in the drawings denote like elements.

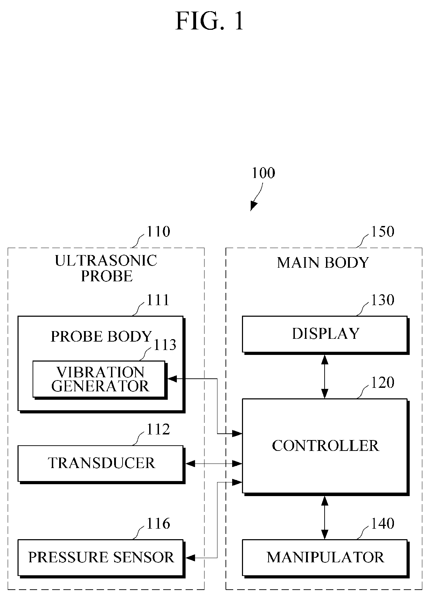

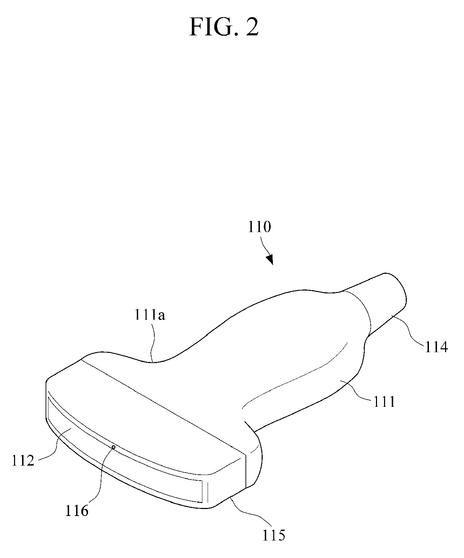

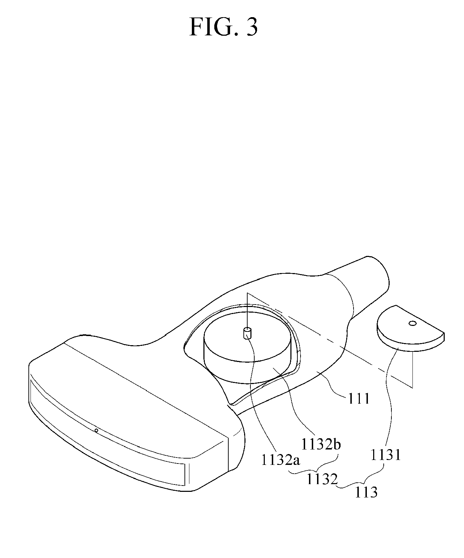

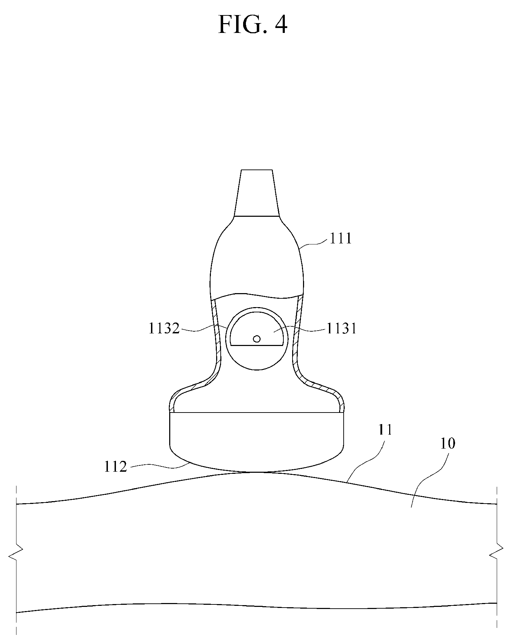

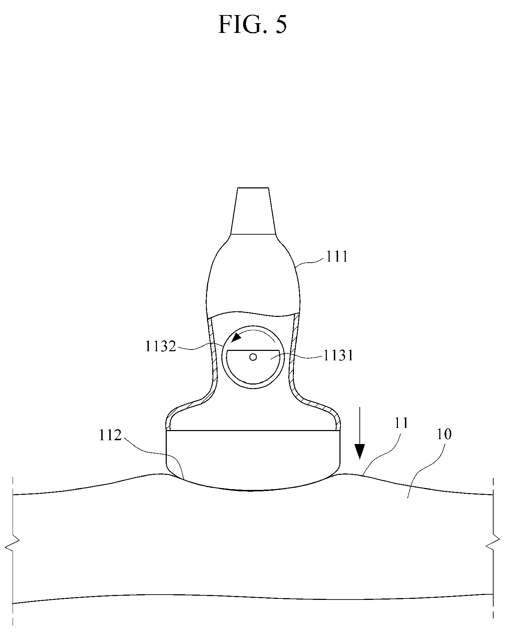

FIG. 1 is a diagram illustrating a configuration of an ultrasonic diagnostic apparatus according to an exemplary embodiment. FIG. 2 is a perspective view illustrating an example of an ultrasonic probe with respect to FIG. 1. FIG. 3 is an exploded perspective view illustrating a vibration generator placed in a probe body with respect to FIG. 2. FIGS. 4 and 5 are diagrams for explaining a procedure for compressing or decompressing a surface of an object using an ultrasonic probe with respect to FIG. 2.

Referring to FIGS. 1 to 5, an ultrasonic diagnostic apparatus 100 includes an ultrasonic probe 110 and a controller 120.

The ultrasonic probe 110 includes a probe body 111, a transducer 112, and a vibration generator 113. The probe body 111 may be structured to have a probe body 111a that is formed in a slender shape enabling a diagnostician to hold the ultrasonic probe 110 with hands comfortably. The probe body 111 has an internal space. The probe body 111 may accommodates the vibration generator 113 installed the internal space thereof. The transducer 112 is fixed to one end portion of the probe body 111.

In a case where the ultrasonic probe 110 is electronically connected to the controller 120 via a cable 114, the probe body 111 may have a structure that allows the cable 114 to come out from the other end portion of the probe body 111. In addition, one end portion of the probe body 111 may be structured to be open. The transducer 112 may be electronically connected to the cable 114 via the open end portion of the probe body 111.

The transducer 112 transmits an ultrasonic signal to internal tissues of an object 10 and receives an ultrasonic signal reflected from the internal tissues of the object 10. The transducer 112 is fixed to one end portion of the probe body 111 and has a contact portion to be in contact with an object's surface 11. The transducer 112 may be installed in an internal space of a protection cap 115 for a purpose of protection. The protection cap 115 may be fixed to one end portion of the probe body 111. The protection cap 115 may have an open portion that is fixed to one end portion of the probe body 111. In addition, the opposite portion of the protection cap 115 may be open, thereby making the contact portion of the transducer 112 exposed. The protection cap 115 may be integratedly formed with the probe body 111.

The vibration generator 113 is accommodated in the probe body 111. The vibration generator 113 vibrates the probe body 111 when the transducer 112 is in contact with the object's surface 11, thereby allowing the transducer 112 to compress or decompress the object's surface 11. For example, the vibration generator 113 may include an eccentric mass 1131 and a rotary actuator 1132.

The eccentric mass 1131 rotates around a rotational axis that is horizontal to the contact portion of the transducer 112. The eccentric mass 1131 may be shaped as a disk with a section that is cut off. A cut-off circumferential surface of the eccentric mass 1131 may be flat, whereas the remaining circumferential surface thereof may be curved. The rotary actuator 1132 makes the eccentric mass 1131 to rotate in an eccentric state. The rotary actuator 1132 may be a rotary motor. Arranged in a horizontal direction to the contact portion of the transducer 112, a driving shaft 1132a of the rotary motor is fixed to an eccentric portion of the eccentric mass 1131. A motor body 1132b of the rotary motor is configured to make the driving shaft 1132a to rotate and is fixed to the probe body 111.

When rotating at 180° each time in response to rotation of the driving shaft 1132a, the eccentric mass 1131 may move between the first and second positions. Herein, the first position may be a position at which a mid-point of the curved circumference of the eccentric mass 1131 may be most spaced apart from the transducer 112, as shown in FIG. 4. The second position may be a position at which a mid-point of the curved circumference of the eccentric mass 1131 may be most closed to the transducer 112, as shown in FIG. 5.

During a procedure in which a mid-point of the curbed circumference of the eccentric mass 1131 oscillates between the first and second positions, the probe body 111 may vibrate. When a mid-point of the curved circumference of the eccentric mass 1131 is moving from the first position to the second position, the object's surface 11 may be compressed by the transducer 112. When a mid-point of the curved circumference of the eccentric mass 1131 is moving from the second position to the first position, the object's surface 11 may be decompressed by the transducer 112.

By obtaining an ultrasonic signal from the transducer 112 when driving the vibration generator 113 to compress or decompress the object's surface 11, the controller 120 may generate an elasticity image. At this point, the controller 120 may generate an elasticity image by obtaining ultrasonic signals that are received, based on information about positions of the eccentric mass 1131 by driving of the rotary actuator 1132, from the transducer 112 at points in time when the transducer 112 compresses and decompresses the object's surface 11.

For example, the vibration generator 113 may include a position detector, such as an encoder (now shown), which is enabled to detect information about an origin position and a current position of the eccentric mass 1131. The controller 120 may generate an elasticity image, by obtaining ultrasonic signals that are received, based on the information detected by an encoder about an origin position or a current position of the eccentric mass 1131, from the transducer 112 at a time when a mid-point of the curved circumference of the eccentric mass 1131 is located at the first position and the second.

The controller 120 may display the elasticity image via the display 130. A manipulation command received from a diagnostician via a manipulator 140 may be input to the controller 120. The controller 120 may be installed to a main body 150 of the ultrasonic diagnostic apparatus 100. The display 130 and the manipulator 140 may be also installed in the min body 150.

The following is an example in which the above-described ultrasonic diagnostic apparatus 100 operates.

When a diagnostician inputs a diagnosis start command to the controller 120 via the manipulator 140, the controller 120 drives the vibration generator 113 to vibrate the probe body 111, thereby vibrating the transducer 112 as well. In this example, the transducer 112 may vibrate at predetermined time intervals with a constant amplitude. In this condition, if the diagnostician makes a contact portion of the transducer 112 to be in contact with the object's surface 11, the object's surface 11 may be periodically compressed and decompressed by the transducer 112. In another example, the controller 120 may control the vibration generator 113 to make the transducer 112 to vibrate aperiodically. In this example, the object's surface 11 may be compressed and decompressed aperiodically.

During the above operation, the controller 120 obtains the first receipt signal that is an ultrasonic signal received from the transducer 112 in response to an ultrasonic signal transmitted to internal tissues of the object 10 at a point in time when the transducer 112 compresses the object's surface 11. In this case, the controller 120 may obtain the first receipt signal at a point in time when the object's surface 11 is most compressed. In addition, the controller 120 obtains the second receipt signal that is an ultrasonic signal received from the transducer 112 in response to an ultrasonic signal transmitted to internal tissues of the object 10 at a point in time when the transducer 112 decompresses the object's surface 11. In this case, the controller 120 may obtain the second receipt signal at a point in time when the object's surface 11 is most decompressed.

Then, the controller 120 generates an elasticity image by combining the first and second receipt signals. A method for generating an elasticity image may be any one of well-known various methods. The controller 120 generates an elasticity image by repeatedly performing the above operation to obtain several numbers of elasticity image data and then average the obtained elasticity image data. Alternatively, the controller 120 may generate an elasticity image by assigning a different weight value to each of the obtained elasticity image data. The controller 120 may display an elasticity image via the display 130. While watching the elasticity image displayed via the display 130, the diagnostician may perform diagnosis on the object 10.

As such, as the vibration generator 113 accommodated in the probe body 111 vibrates the ultrasonic probe 110, the object's surface is compressed or decompressed, and thus, more uniform elasticity images may be obtained in real time, compared to a case where a diagnostician manually applies a static force or vibration to the ultrasonic probe 110. In addition, it is possible to improve repeatability, reproducibility, and quality of an elasticity image.

Meanwhile, as shown in FIGS. 1 and 2, the ultrasonic probe 110 may include a pressure sensor 116. The pressure sensor 116 may be installed at the contact portion of the transducer 112 so that the pressure sensor 116 is allowed to detect pressured applied on the object's surface 11. Pressure information detected by the pressure sensor 116 may be provided to the controller 120.

The controller 120 generates an elasticity image, by determining, based on the information detected by the pressure sensor 116, at points in time when maximum and minimum pressure are applied on the object's surface 11 and then obtaining an ultrasonic signal received from the transducer 112 at each of the determined points in time. Alternatively, the controller 120 may generate an elasticity image, by obtaining an ultrasonic signal that are received, based on information detected by the pressure detector 116, from the transducer 112 at points in time when two different pressures are applied, wherein magnitude of the two different pressures applied is smaller than the maximum pressure but greater than the minimum pressure. The pressure sensor 116 may be any one of well-known various pressure sensors as far as it is capable of performing the above operation.

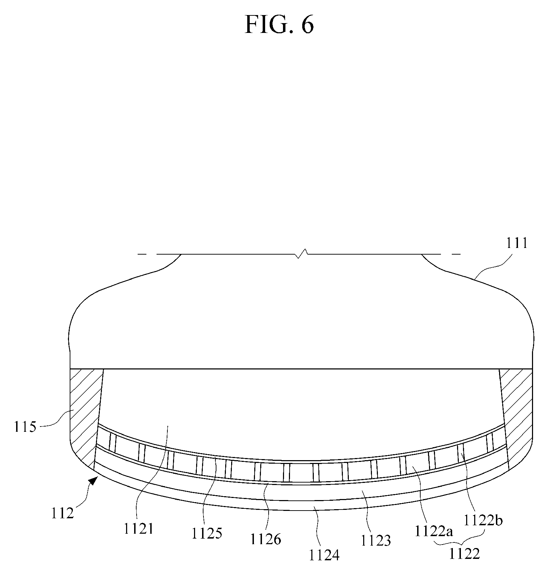

As illustrated in FIG. 6, the transducer 112 may be configured to include a backing material 1121, a piezo-electric layer 1122, a matching layer 1123, and an acoustic lens 1124, wherein the piezo-electric layer 1122, the matching layer 1123, and the acoustic lens 1124 are layered on one side of the backing material 1121 in the order named.

The backing material 1121 may have absorbing properties. The backing material 1121 may reduce a pulse width by refraining vibration of the piezo-electric layer 1122 layered thereon, and may avoid image distortion by preventing ultrasonic waves from unnecessarily spreading below the piezo-electric layer 1122. In a case where the transducer 112 is a convex array type, the backing material 1121 may have a convexly curved surface of a predetermined curvature, the curved surface on which the piezo-electric layer 1122 is layered.

The piezo-electric layer 1122 generates an ultrasonic signal by resonating in response to application of voltage, while generating an electric signal in response to receipt of an ultrasonic signal. The piezo-electric layer 1122 may have a plurality of spaced-apart piezoelectric elements 1122a of predetermined thickness, which are arranged on the curved surface of the backing material 1121. A filler material 1122b is filled in a space between each two spaced-apart piezoelectric elements 1122a, thereby fixing the piezoelectric elements 1122a.

The matching layer 1123 may reduce difference in acoustic impedance between the piezoelectric elements 1122a and the object 10. An acoustic lens 1124 is used to focus ultrasonic waves generated from the piezoelectric elements 1122a. A surface of the acoustic lens 1124, except the sides thereof, is exposed via an end portion of the protection cap 115, so that the surface of the acoustic lens 1124 may function as a contact portion to be in contact with the object's surface 11. The acoustic lens 1124 is positioned on top of the matching layer 1123, both of which are of predetermined thickness, and the matching layer 1123 and the acoustic lens 1124 are stacked on the curved surface of the piezo-electric layer 1122 in a curved form in the order of the piezo-electric layer 1122, the matching layer 1123, and the acoustic lens 1124.

In another example, the transducer 112 may be a linear array type in which a plurality of piezoelectric elements 1122a of predetermined thickness are arranged on a flat surface of the backing material 1121, and therefore, aspects of the present disclosure are not limited thereto.

Then, a first electrode part 1125 may be arranged between the backing material 1121 and the piezo-electric layer 1122. The first electrode part 1125 may be configured as a flexible printed circuit board, wherein first electrodes corresponding to the respective piezoelectric elements 1122a are formed on one side of the flexible printed circuit board. In addition, a second electrode part 1126 may be arranged between the piezo-electric layer 1122 and the matching layer 1123.

The second electrode part 1126 may be configured as a flexible printed circuit board, one side of which second electrodes corresponding to the respective piezoelectric elements 1122a are formed. In a case where the first electrodes of the first electrode part 1125 function as signal electrodes used for transmission/receipt of an electric signal, the second electrodes of the second electrode part 1126 may function as ground electrodes. Both the first and second electrode parts 1125 and 1126 may be electrically connected to the cable 114 via a connector.

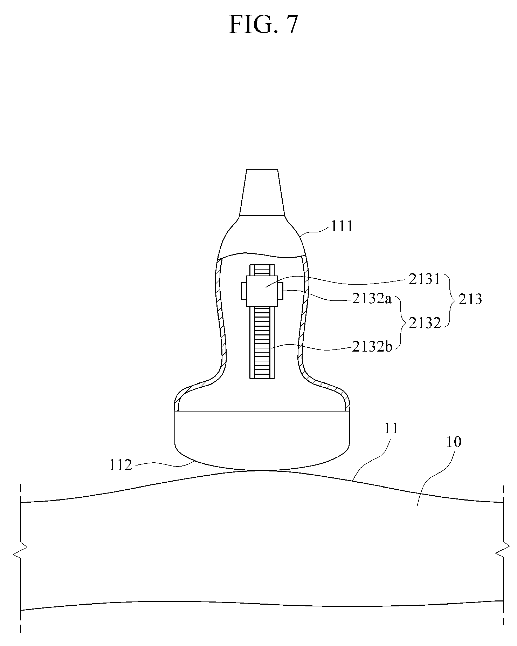

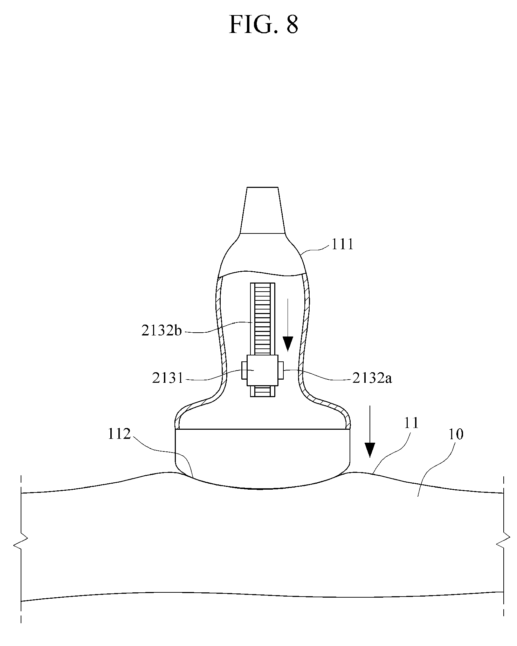

FIG. 7 is a partial sectional view illustrating another example of a vibration generator. FIG. 8 is a partial sectional view illustrating an example in which an object's surface is compressed by a vibration generator with respect to FIG. 7.

Referring to FIGS. 7 and 8, a vibration generator 213 may include a mass 2131 and a linear actuator 2132. The mass 2131 slides back and forth in a direction toward and away from the contact portion of the transducer 112. The linear actuator 2132 makes the mass 2131 to slide back and forth. The linear actuator 2132 may be a linear motor. The linear motor includes a mover 2132a and a stator 2132b. The mover 2132a may include a permanent magnetic and the stator 2132b may include a coil for receiving electric currents, or vice versa. Once electric currents are applied to a coil positioned within a magnetic field of a permanent magnetic, the mover 2132a linearly moves with respect to the stator 2132b in response to a Lorentz force. The stator 2132b may include a rail that guides linear movement of the mover 2132a.

The mover 2132a may have the mass 2131 installed therewith, so that the mover 2132a may move together with the mass 2131. Another possible example is that the mass 2131 is omitted and the mover 2132a functions as the mass 2131. Arranged to enable the mover 2132a to slide back and forth in a direction toward and away from the contact portion of the transducer 112, the stator 2132b may be fixed to the probe body 111.

The mass 2131 may move between the first and second positions in response to sliding movement of the mover 2132a. In this case, the first position may correspond to a position at which the mass 2131 is spaced farthest apart from the transducer 112, as shown in FIG. 7. The second position may correspond to a position at which the mass 2131 is closest to the transducer 112, as shown in FIG. 8. As sliding back and forth between the first and second positions, the mass 2131 may make the probe body 111 to vibrate. When the mass 2131 moves from the first position to the second position, the object's surface 11 may be compressed by the transducer 112. When the mass 2131 moves from the second position to the first position, the object's surface 11 may be decompressed by the transducer 112.

The controller 120 may generate an elasticity image, by obtaining ultrasonic signals that are received, based on information about a position of the mass 2131 by driving of the linear actuator 2132, from the transducer 112 at points in time when the transducer 112 compresses and decompresses the object's surface 11. For example, a linear motor may include a position detector (now shown) that detects a position of the mover 2132a. The controller 120 may obtain information about a position of the mass 2131 based on the information detected by the position detector about a position of the mover 2132a. The controller 120 may generate an elasticity image by obtaining at ultrasonic signals that are received, based on information about a position of the mass 2131, from the transducer 112 at points in time when the mass 2131 moves to be located at the first and second positions. Alternatively, the controller 120 may generate an elasticity image by obtaining ultrasonic signals received from the transducer 112 at points in time when the mass 2131 is located at two specific positions between the first and the second positions.

In another example, a linear actuator may be configured to include a guide that guides linear movement of the mass 2131, a ball screw coupled to the mass 2131, and a rotary motor that makes the ball screw to rotate, or configured differently in various ways, and thus, the linear actuator is not limited to the above-described example.

It will be apparent to those skilled in the art that various modifications and variation can be made in the present invention without departing from the spirit or scope of the invention. Thus, it is intended that the present invention cover the modifications and variations of this invention provided they come within the scope of the appended claims and their equivalents.

## Claims

1. An ultrasonic probe comprising:
a probe body;
a transducer fixed to one end portion of the probe body and configured to transmit and receive an ultrasonic signal and have a contact portion to be in contact with a surface of an object; and
a vibration generator accommodated in the probe body and configured to vibrates the probe body when the transducer is in contact with the surface of the object, thereby allowing the transducer to compress and decompress the surface of the object.

2. The ultrasonic probe of claim 1, wherein the vibration generator comprises:
an eccentric mass configured to rotate around a rotation axis that is horizontal to the contact portion of the transducer; and
a rotary actuator configured to make the eccentric mass to rotate in an eccentric state.

3. The ultrasonic probe of claim 1, wherein the vibration generator comprises:
a mass configured to slide back and forth in a direction toward and away from the contact portion of the transducer; and
a linear actuator configured to make the mass to slide back and forth

4. The ultrasonic probe of claim 1, further comprising:
a pressure sensor installed at the contact portion of the transducer and configured to detect pressure applied by the transducer on the surface of the object.

5. An ultrasonic diagnostic apparatus comprising:
an ultrasonic probe comprising:
a probe body;
a transducer is fixed to one end portion of the probe body and configured to transmit and receive an ultrasonic signal and have a contact portion to be in contact with a surface of an object; and
a vibration generator accommodated in the probe body and configured to vibrates the probe body when the transducer is in contact with the surface of the object, thereby allowing the transducer to compress and decompress the surface of the object,
a controller configured to generate an elasticity image by obtaining an ultrasonic signals received from the transducer at a time when driving the vibration generator to compress or decompress the surface of the object.

6. The ultrasonic diagnostic apparatus of claim 5, further comprising:
a pressure sensor installed at the contact portion of the transducer, thereby enabled to detect pressure applied by the transducer on the surface of the object.

7. The ultrasonic diagnostic apparatus of claim 6, wherein the controller is further configured to generate an elasticity image, by obtaining ultrasonic signals that are received, based on information detected by the pressure sensor, from the transducer at points in time when maximum and minimum pressure are applied on the surface of the object.

8. The ultrasonic diagnostic apparatus of claim 5, wherein the vibration generator comprises:
an eccentric mass configured to rotate around a rotation axis that is horizontal to the contact portion of the transducer; and
a rotary actuator configured to make the eccentric mass to rotate in an eccentric state.

9. The ultrasonic diagnostic apparatus of claim 8, wherein the controller is configured to generate an elasticity image, by obtaining ultrasonic signals that are received, based on information of a position of the eccentric mass by driving of the rotary actuator, from the transducer at points in time when the surface of the object is compressed and decompressed by the transducer.

10. The ultrasonic diagnostic apparatus of claim 5, wherein the vibration generator comprises:
a mass configured to slide back and forth in a direction toward and away from the contact portion of the transducer; and
a linear actuator configured to make the mass to slide back and forth.

11. The ultrasonic diagnostic apparatus of claim 10,
wherein the controller is further configured to generate an elasticity image, by obtaining ultrasonic signals that are received, based on information about a position of the mass by driving of the linear actuator, from the transducer at points in time when the surface of the object is compressed and decompressed by the transducer.
